# EUROPEAN PATENT SPECIFICATION

(11) **EP 3 758 739 B1**
(45) Date of publication and mention of the grant of the patent: **15.05.2024**
(21) Application number: 19708487.4
(22) Date of filing: 28.02.2019
(51) Int. Cl.: A61K 38/57, A61P 9/10

(54) **TREATMENT AND PREVENTION OF PRE-ECLAMPSIA**
BEHANDLUNG UND PRÄVENTION VON PRÄEKLAMPSIE
TRAITEMENT ET PRÉVENTION DE PRÉ-ÉCLAMPSIE

(30) Priority: 28.02.2018 EP 18159064
(43) Date of publication of application: 06.01.2021
(73) Proprietor: Pharming Intellectual Property B.V., 2333 CR Leiden (NL)
(72) Inventor: DE VRIES, Sijmen, 2333 CR Leiden (NL); GIANNETTI, Bruno, 2333 CR Leiden (NL)
(74) Representative: Nederlandsch Octrooibureau
(86) International application number: PCT/EP2019/055001
(87) International publication number: WO 2019/166556

(56) References cited:
- WO-A1-92/22320
- WO-A1-2004/100982
- WO-A2-01/57079
- HALBMAYER W M ET AL: "C1-esterase inhibitor in uncomplicated pregnancy and mild and moderate preeclampsia", THROMBOSIS AND HAEMOSTASIS, SCHATTAUER GMBH, DE, vol. 65, no. 2, 12 February 1991 (1991-02-12), pages 134-138, XP009505109, ISSN: 0340-6245
- TERESA CABALLERO ET AL: "Management of hereditary angioedema in pregnant women: a review", INTERNATIONAL JOURNAL OF WOMEN'S HEALTH, 1 September 2014 (2014-09-01), page 839, XP055472270, DOI: 10.2147/IJWH.S46460
- Admin: "Pharming reports positive Phase II study results of RUCONEST to treat hereditary angioedema - Clinical Trials Arena", , 17 July 2016 (2016-07-17), XP93034753, Retrieved from the Internet: URL:https://www.clinicaltrialsarena.com/ne ws/newspharming-reports-positive-phase-ii- study-results-ruconest-treat-hereditary-an gioedema-4951993/ [retrieved on 2023-03-27]

## Description

### Field of the invention

The present invention relates to the field of medicine, specifically to the prevention of pre-eclampsia.

### Background of the invention

Pre-eclampsia (PE) is a placental disease [1] characterized by insufficiency of the uteroplacental circulation [2], and which affects 10-12% of all pregnancies and is a major factor in the perinatal mortality rate. Pre-eclampsia is a severe complication of human pregnancy characterized by development of hypertension and proteinuria and it affects maternal and foetal morbidity and mortality worldwide [3]. In developed countries, pre-eclampsia has been reported to complicate 1 to 4% of all pregnancies [3] while in developing countries the prevalence can be as high as 17% [4]. In developing countries hypertensive disorders of pregnancy heavily contribute to all maternal and foetal deaths [5]. There is evidence that one or more placental-derived factors are released into the maternal circulation which either directly or indirectly cause maternal endothelial dysfunction and ensuing maternal problems with activation of the clotting system increased vascular permeability and ischemia in maternal organs secondary to vasoconstriction [6]. It has been postulated that administration of a P-type IPG antagonist could be effective in the treatment of pre-eclampsia (WO9810791). To date however, there is no treatment that is satisfactorily effective for the prevention or treatment of pre-eclampsia except for delivery of the baby.

WO 2004/100982 A1 relates to the use of a C1 inhibitor with a modified carbohydrate structure in the treatment or prevention of pre-eclampsia. WO 01/57079 A2 relates to the use of C1 inhibitor in the prophylactic treatment of pre-eclampsia (among other possible C1 inhibitor deficiencies to be treated). WO 92/22320 A1 relates to a method of treating or preventing pre-eclampsia by administering C1-INH to the patient. TERESA CABALLERO ET AL: "Management of hereditary angioedema in pregnant women: a review", INTERNATIONAL JOURNAL OF WOMEN'S HEALTH, 1 September 2014 (2014-09-01), page 839 reports on C1-INH activity and antigen reductions in association with pregnancy.

### Summary of the invention

The invention provides for a compound for use in the prevention of pre-eclampsia in a subject at risk of pre-eclampsia, wherein said compound is a complement inhibitor.

### Detailed description of the invention

The inventors arrived at the surprising finding that inhibition of the complement system can be effective for the prevention of pre-eclampsia. The invention is defined in the claims.

Accordingly, in a first aspect, the invention provides for a compound for use in the prevention of pre-eclampsia in a subject at risk of pre-eclampsia, wherein said compound is a complement inhibitor. Herein, the compound for use is referred to as the compound according to the invention. In all embodiments of the invention, a complement inhibitor is construed as any compound that is capable of inhibiting the complement pathway to at least some extent, such as one step within the pathway. Pre-eclampsia is construed herein as defined in the background section here above. The compound according to the invention may be any compound, it may be a small molecule, a protein, peptide, an antibody, an enzyme, an enzyme inhibitor such as a protease inhibitor or a chelator. Prevention of pre-eclampsia is herein construed as a delay of the onset of pre-eclampsia and/or a significant reduction of pre-eclampsia when the onset of pre-eclampsia occurs in a person at risk of pre-eclampsia.

In the embodiments of the invention, the complement inhibitor can be a C1 esterase inhibitor. The C1 esterase inhibitor may be any C1 esterase inhibitor known to the person skilled in the art. In an embodiment, the C1 esterase inhibitor is an antibody directed against (human) C1 inhibitor. In the embodiments of the invention, the C1 esterase inhibitor can be a plasma-derived C1 esterase inhibitor. In the embodiments of the invention, the C1 esterase inhibitor can be a recombinant C1 esterase inhibitor, preferably a C1 esterase inhibitor having an amino acid sequence that is substantially identical to the amino acid sequence of human plasma-derived C1 esterase inhibitor. The recombinant C1 esterase inhibitor can be any recombinant C1 esterase inhibitor known the person skilled in the art. It may be produced recombinantly in microbial cells, such as tissue culture cells. The tissue culture cell can be a mammalian tissue culture cell, such as a Chinese Hamster Ovarian (CHO) cell or a human tissue culture cell (see e.g. WO2016/081889). The recombinant C1 esterase inhibitor can be produced in transgenic animals, such as in a transgenic non-human mammal, preferably a mouse, goat, bovine, sheep, porcine or an animal from the order *Lagomorpha,* such as a *Leporadae,* including a rabbit. In an embodiment, the recombinant C1 esterase inhibitor is one produced according to the methods in WO01/57079.

In the embodiments of the invention, the C1 esterase inhibitor can be a modified C1 esterase inhibitor as compared to human plasma-derived C1 esterase inhibitor. It can be modified to modulate the plasma half-life of the C1 esterase inhibitor. A specific modified C1 esterase inhibitor is conjugated to enhance the plasma half-life. An exemplary conjugated C1 esterase inhibitor to enhance half-life is a conjugated C1 esterase inhibitor according to WO2017/176798, such as a polysialic acid (PSA)-conjugated C1 esterase inhibitor, more preferably a polyethylene glycol (PEG)-conjugated C1 esterase inhibitor. The modification of the C1 esterase inhibitor can be a modified carbohydrate structure as compared to human plasma-derived C1 esterase inhibitor. A specific modified C1 esterase inhibitor has a reduced level of terminal sialic acid residues as compared to plasma derived C1 esterase inhibitor, wherein said reduced level of terminal sialic acid residues may result in a reduction of plasma half-life to less than 6 hours. A specific C1 esterase inhibitor having a reduced level of terminal sialic acid residues as compared to plasma derived C1 esterase inhibitor is a C1 esterase inhibitor according to WO01/57079, WO2004/100982 and WO2007/073186.

The compound according to the invention can be administered as such and can be administered comprised in a pharmaceutical composition. The pharmaceutical composition can comprise a pharmaceutically accepted excipient and/or can comprise a further pharmaceutical compound. The compound according to the invention may be administered by any means known to the person skilled in the art, such as but not limited, to intravenous, transdermal and subcutaneous administration. Intravenous administration is extensively described in WO01/57079, WO2004/100982 and WO2007/073186. Subcutaneous administration is preferably performed as in WO2014/145519, US9616111B2 and EP2968434B1.

In the embodiments of the invention, the compound according to the invention can be administered to the subject at least twice, three, four, five, six or seven times a week or can be administered, every other day, daily, or twice a day.

When the compound according to the invention is a C1 esterase inhibitor, the compound can be administered in a dose ranging from 25 units/kg body weight to 100 units/kg body weight per administration, preferably ranging from 50 units/kg body weight to 100 units/kg body weight per administration. Per administration the dose can be 25 units/kg body weight, 50 units/kg body weight, 100 units/kg body weight. The total dose per administration can be 1000 units, 1400 units, 1500 units, 2000 units, 2100 units, 2800 units, 3000 units, 3500 units, 4000 units, 4200 units, 4500 units, 4900 units, 5000 units, 5600 units, 6000 units, 6300 units, 7000 units, 7500 units, 8000 units, 8400 units or 9000 units C1 inhibitor.

In the embodiments of the invention, the subject can be a pregnant mammal, preferably a pregnant human.

The diagnosis of a risk of pre-eclampsia can be made by any means and assay known to the person skilled in the art. The diagnosis may e.g. be made by assessing whether there is occurrence of hypertension and proteinuria. The diagnosis can be made by measurement of P-type inositolphosphoglycans (P-type IPG) in a bodily fluid, such as in blood or urine.

Accordingly, the invention provides for a compound for use according to the invention, wherein the subject is diagnosed with being at risk of pre-eclampsia by measurement of P-type inositolphosphoglycans (P-type IPG) in a bodily fluid, such as in blood or urine. In an embodiment, the invention provides for a compound for use according to the invention, wherein the subject is diagnosed with of being at risk of pre-eclampsia by measurement of P-type inositolphosphoglycans (P-type IPG) in a bodily fluid, such as in blood or urine. Preferably, the P-type inositolphosphoglycans (P-type IPG) is placenta-derived P-type inositolphosphoglycans (P-type IPG). In the embodiments of the invention, the measurement can be performed by any means known to the person skilled in the art, such as by using the method as described in WO9810791. Preferably, the measurement is performed according to experiments A-1, A-2, A-5 and A-6 of WO9810791. For reference, the here mentioned parts of WO9810791 are copied here below.

The activity of P- and A-type IPGs in urine and placental extracts were studied using specific bioassay procedures. IPG P-type was determined using the activation of PDH phosphatase [7]. The PDH complex and PDH phosphatase (metal-dependent form) were prepared from beef heart as described by Lilley et al. [7] and the assay of the activation of the phosphatase was performed by the spectrophotometric variant of the two-stage system described by these authors. This assay is considered to be a characteristic feature of IPG P-type (see Lamer et al. [8]). IPG A-type was determined by the stimulation of lipogenesis as measured by the incorporation of [U¹⁴C] glucose into the lipids of adipocytes isolated from epididymal fat pads by the method of Rodbell [9]. A high degree of specificity for IPG A-type was found for this bioassay.

A straight line relationship between added IPGs and the stimulation of PDH phosphatase activity (IPG P-type) and lipogenesis in intact adipocytes (IPG A-type) was obtained; this relationship held at least up to a stimulation of-1-250%. These observations provided a basis for a unit to be defined and used for the purpose of comparison of yields of IPGs from different tissues and urine samples. Linearity between IPG added and the percentage change in response, has been observed by others (see Lilley et al. [7] and Newman et al. [10]), although Asplin et al. [11] did not show linearity in their study on IPGs in human urine from normal and diabetic subjects, an effect which was particularly marked with the IPG A-type (pH 1.3 fraction).

Extraction of IPG P-type and IPG-A type from urine was performed as described by Asplin et. al. [11]. The final fractions were freeze dried and stored at - 20°C. For use, the IPG fractions were resuspended in water, immediately before assay, so that 10µl of redissolved IPG corresponded to 10ml urine.

In view of the possibility that high, and varying, amounts of IPGs might be excreted in the different groups of pregnant and pre-eclamptic subjects, and in order to ensure that the capacity of the resin was well in excess of the load applied, preliminary test runs were made to determine the optimal ratio of resin to starting urine volume. Linearity of recovery was obtained up to 100ml urine per 18g resin. In the present study, the ratio of 30ml urine to 18g resin was maintained to allow for variation in IPG content.

Expression of results: A unit of IPG is defined as the amount causing a 50% activation in the basal level of the test system.

The yield of IPGS in urine is given on three different bases:
(i) Percentage stimulation of the test system by 10µl final urine extract (Col 1), allowing direct comparison with data of Asplin et. al. [11]
(ii) Units of IPG per 1 mmol creatinine.
(iii) Units of IPG found in a sample of a 24 hour collection or urine; i.e.: the total daily output at that stage of gestation.

Unless otherwise indicated each embodiment as described herein may be combined with another embodiment as described herein.

### Definitions

In this document and in its claims, the verb "to comprise" and its conjugations is used in its nonlimiting sense to mean that items following the word are included, but items not specifically mentioned are not excluded. In addition the verb "to consist" may be replaced by "to consist essentially of' meaning that a product or a composition or a nucleic acid molecule or a peptide or polypeptide of a nucleic acid construct or vector or cell as defined herein may comprise additional component(s) than the ones specifically identified; said additional component(s) not altering the unique characteristic of the invention. In addition, reference to an element by the indefinite article "a" or "an" does not exclude the possibility that more than one of the elements is present, unless the context clearly requires that there be one and only one of the elements. The indefinite article "a" or "an" thus usually means "at least one".

Herein, one unit (U) of C1 esterase inhibitor is the amount of C1 esterase inhibitor present in 1 milliliter of human plasma. One such unit corresponds to approximately 275 microgram plasma-derived C1 esterase inhibitor.

The following examples are offered for illustrative purposes only, and are not intended to limit the scope of the present invention in any way.

### Examples

### Example 1

C1 esterase inhibitor from transgenic rabbits prepared as in WO01/57079 (Ruconest^{™}, Pharming, the Netherlands) is administered on daily basis in a 50 units/kg bodyweight dose to pregnant human subjects suffering from early-onset pre-eclampsia (<34 weeks gestational age) or from late-onset pre-eclampsia (>34 weeks gestational age). A control group receives no treatment except for the state of the art hospitalization. On average, the treated groups demonstrate significant positive effects of the treatment as demonstrated by lower blood pressure and lower proteinuria.

### Example 2

C1 esterase inhibitor from transgenic rabbits prepared as in WO01/57079 (Ruconest^{™}, Pharming, the Netherlands) is administered on daily basis in a 50 units/kg bodyweight dose to pregnant human subjects at risk of pre-eclampsia (>34 weeks gestational age). A control group receives no treatment except for the state of the art hospitalization. On average, the treated group demonstrates significant positive effects of the treatment as demonstrated by no or later onset of pre-eclampsia and lower blood pressure and lower proteinuria when pre-eclampsia does occur.

### Example 3

### SYNOPSIS PRE-ECLAMPSIA CLINICAL STUDY

**Study Title:** A Phase I/II, Open Label, Proof of Concept Study to investigate Tolerability and Safety of Treatment with Recombinant Human C1 Inhibitor (conestat alfa) in Patients with pre-eclampsia.

### Study Phase: Phase I/II, proof of concept study

**Number of Patients:** Up to 30 patients can be enrolled. Recruitment will stop once 20 patients completed the treatment period or 30 patients have been enrolled whichever comes first. Already enrolled patients will complete the study as per protocol.

### Rationale:

The current trial intends to evaluate the tolerability and safety of recombinant human C1 esterase inhibitor (rhC1 INH) conestat alfa - in the patient with pre-eclampsia and further explore the efficacy of such a treatment.

### Objectives:

### Primary:

To evaluate the tolerability and safety of the treatment with rhC1INH (conestat alfa) on top of Standard Care, for patients with pre-eclampsia.

### Secondary:

To evaluate the efficacy of treatment with rhC1INH (conestat alfa) on top of Standard Care, for patients with pre-eclampsia.

### Exploratory:

To further characterize the clinical safety of treatment with rhC1INH (conestat alfa) on top of Standard Care, for patients with pre-eclampsia by measuring evolution of laboratory parameters like platelet count, LDH, ALAT, ASAT, Kreat, Hb, Ht, urine protein, urine kreatinine,

To identify changes in biomarkers of pre-eclampsia (PIGF, VEGF, sFlt-1, IPG, podocytes level) following rhC1INH (conestat alfa) treatment.

To analyze complement activation and other immune characteristics in the mother's blood and urine (C4, C1q, C5b and factor H) and in the placenta and cord blood (C4, C1q, C5b and factor H, leucocyte count and FACS analysis)
To evaluate immune system in the baby's blood after birth by measuring complement activation (C4, C1q, C5b, factor H) leucocyte count and performing a FACS analysis.

To evaluate plasma pharmacokinetic profile of rhC1 INH (conestat alfa) in pregnant women.

### Study design:

This is an open label, proof of concept study on the treatment with rhC1INH (conestat alfa) of patients with pre-eclampsia between 27-34 weeks gestation. The study will consist of a screening visit, an enrolment visit, a treatment period and a follow-up period.

### Study population:

Up to 30 pregnant women between 27-34 weeks of gestation referred to hospital care centers for management of their pregnancy with pre-eclampsia, fulfilling the inclusion criteria, are eligible for participation in the study:

### Intervention:

Twice weekly open label intravenous treatment with conestat alfa at a dose of 50 units/kg (based on body weight at start of treatment) up to a maximum of 4200 units on top of Standard Care.

### Main study parameters/endpoints:

### Primary endpoint:

- Incidence and severity of adverse events
- Number and percentage of patients who discontinue investigational product or withdraw from the study

### Secondary endpoints:

- Time from start of conestat alfa to day of delivery
- Proportion of patients reaching gestation week 37

### Explorative endpoints:

### Mother:

- Biochemistry and hematology parameters over time, including safety parameters like platelet count, LDH, ALAT, ASAT, creatinine, Hb, Ht.
- Urine protein and creatinine level
- Number of women who develop HELLP syndrome, eclampsia.
- Number of women with placental abruption
- Plasma concentration of C1INH - pharmacokinetic profile analysis
- Biomarkers in blood sFlt-1, VEGF and PIGF
- Biomarkers in urine: IPG and podocyte levels
- Gestational age at birth
- Incidence of admission to a neonatal intensive care unit as applicable
- Time spent in the neonatal intensive care unit
- Normalization of uterine Doppler flow profiles
- Normalization of Doppler profiles of the Umbilical artery and/or medial cerebral artery
- Quantification of complement activation in placental tissue (C1q)
- Quantification of complement activation in maternal blood and urine samples (C4, C1q, C5b, factor H)
- Proportion of patients reaching gestation week 34, or 30

### Baby:

- Birthweight
- Delivery of healthy baby, defined as: a term baby, normal weight (= per country/part of the world), normal APGAR score, no congenital abnormalities, normal head circumference
- Incidence of neonatal:
   ∘ Necrotizing enterocolitis
   ∘ Respiratory distress syndrome,
   ∘ Cerebral hemorrhage,
   ∘ Grade 3 - 4 intraventricular hemorrhage,
   ∘ Bronchopulmonary dysplasia
- Normal Immune system in the baby's blood and cord blood by measuring complement activation (C4, C1q, C5b, factor H), leucocyte count and a performing a FACS analysis
- Normal pediatric echocardiography after delivery

### Efficacy analyses:

Efficacy analyses will be performed for the following:
- Time from start of treatment to delivery
- Patients reaching gestation week 37

### Pharmacokinetic profile Analyses

The mean plasma concentrations of C1INH over time will be plotted and analyzed visually.

### Biomarkers for PE

- Biomarkers for pre-eclampsia (sFlt-1, PIGF, VEGF, podocytes levels and IPG)
- Quantification of complement activation in placental tissue and cord blood (C4, C1q, C5b, factor H)
- Quantification of complement activation in maternal blood and urine samples (C4, C1q, C5b, factor H)
- Quantification of complement activation in baby's blood (C4, C1q, C5b, factor H) at 3 months follow up visit

### References

1. Redman, C.W.G., 1991. Pre-eclampsia and the placenta. Placenta, 12(4), pp.301-308.
2. Robertson, W.B., Brosens, I. and Dixon, H.G., 1967. The pathological response of the vessels of the placental bed to hypertensive pregnancy. The Journal of Pathology, 93(2), pp.581-592.
3. Steegers, E.A., von Dadelszen, P., Duvekot, J.J. and Pijnenborg, R., 2010. Pre-eclampsia. The Lancet, 376(9741), pp.631-644.
4. Osungbade, K.O. and Ige, O.K., 2011. Public health perspectives of preeclampsia in developing countries: implication for health system strengthening. Journal of pregnancy, 2011.
5. The world health report 2005 - make every mother and child count. WHO, Geneva. Available at: www.who.int/whr/2005/en/.
6. Romero, G., 1991. Inositolglycans and cellular signalling. Cell biology international reports, 15(9), pp.827-852.
7. Lilley, K., Zhang, C., Villar-Palasi, C., Larner, J. and Huang, L., 1992. Insulin mediator stimulation of pyruvate dehydrogenase phosphatases. Archives of biochemistry and biophysics, 296(1), pp.170-174.
8. Larner, J., Huang, L.C., Suzuki, S., Tang, G., Zhang, C., Schwartz, C.F.W., Romero, G., Luttrell, L. and Kennington, A.S., 1989. Insulin mediators and the control of pyruvate dehydrogenase complex. Annals of the New York Academy of Sciences, 573(1), pp.297-305.
9. Rodbell, M., 1964. The metabolism of isolated fat cells. Comprehensive Physiology.
10. Newman, J., Armstrong, J.M. and Bornstein, J., 1985. Assay of insulin mediator activity with soluble pyruvate dehydrogenase phosphatase. Endocrinology, 116(5), pp.1912-1919.
11. Asplin, I., Galasko, G. and Larner, J., 1993. Chiro-inositol deficiency and insulin resistance: a comparison of the chiro-inositol-and the myo-inositol-containing insulin mediators isolated from urine, hemodialysate, and muscle of control and type II diabetic subjects. Proceedings of the National Academy of Sciences, 90(13), pp.5924-5928.

## Claims

1. A compound for use in the prevention of pre-eclampsia in a pregnant mammal at risk of pre-eclampsia, wherein said compound is a C1 esterase inhibitor and wherein the compound is administered in a dose ranging from 25 units/kg body weight to 100 units/kg body weight per administration to the pregnant mammal at least twice a week.

2. A compound for use according to claim 1, wherein the C1 esterase inhibitor is a recombinant C1 esterase inhibitor, such as a C1 esterase inhibitor having an amino acid sequence that is substantially identical to the amino acid sequence of human plasma-derived C1 esterase inhibitor.

3. A compound for use according to claim 2, wherein the recombinant C1 esterase inhibitor is produced in a transgenic non-human mammal, such as a mouse, goat, bovine, sheep, porcine or an animal from the order *Lagomorpha,* such as a *Leporadae,* including a rabbit.

4. A compound for use according to claim 3, wherein the C1 esterase inhibitor has a reduced level of terminal sialic acid residues as compared to plasma derived C1 esterase inhibitor, wherein said reduced level of terminal sialic acid residues results in a plasma half-life of less than 6 hours.

5. A compound for use according to any one of claims 1 - 4, wherein the compound is administered in a dose ranging from 50 units/kg body weight to 100 units/kg body weight per administration.

6. A compound for use according to any one of the preceding claims, wherein the pregnant mammal is a pregnant human.

7. A compound for use according to any of the preceding claims, wherein the pregnant mammal is diagnosed of being at risk of pre-eclampsia by measurement of P-type inositolphosphoglycans (P-type IPG) in a bodily fluid, such as in blood or urine.

8. A compound for use according to any of the preceding claims, wherein the pregnant mammal is diagnosed of being at risk of pre-eclampsia by measurement of placenta-derived P-type inositolphosphoglycans (P-type IPG) in a bodily fluid, such as in blood or urine.

## Patentansprüche

1. Verbindung zur Verwendung bei der Vorbeugung von Präeklampsie bei einem trächtigen Säugetier mit dem Risiko einer Präeklampsie, wobei die Verbindung ein C1-EsteraseInhibitor ist und wobei die Verbindung in einer Dosis im Bereich von 25 Einheiten/kg Körpergewicht bis 100 Einheiten/kg Körpergewicht pro Verabreichung an das trächtige Säugetier mindestens zweimal pro Woche verabreicht wird.

2. Verbindung zur Verwendung nach Anspruch 1, wobei der C1-Esterase-Inhibitor ein rekombinanter C1-Esterase-Inhibitor ist, wie ein C1-Esterase-Inhibitor mit einer Aminosäuresequenz, die im Wesentlichen identisch mit der Aminosäuresequenz des aus menschlichem Plasma stammenden C1-Esterase-Inhibitors ist.

3. Verbindung zur Verwendung nach Anspruch 2, wobei der rekombinante C1-EsteraseInhibitor in einem transgenen nicht-menschlichen Säugetier, wie einer Maus, einer Ziege, einem Rind, einem Schaf, einem Schwein oder einem Tier aus der Ordnung *Lagomorpha,* wie *Leporidae,* einschließlich eines Kaninchens, produziert wird.

4. Verbindung zur Verwendung nach Anspruch 3, wobei der C1-Esterase-Inhibitor im Vergleich zu einem aus Plasma stammenden C1-Esterase-Inhibitor einen verringerten Gehalt an terminalen Sialinsäure-Resten aufweist, wobei der verringerte Gehalt an terminalen Sialinsäureresten zu einer Plasma-Halbwertszeit von weniger als 6 Stunden führt.

5. Verbindung zur Verwendung nach einem der Ansprüche 1 - 4, wobei die Verbindung in einer Dosis im Bereich von 50 Einheiten/kg Körpergewicht bis 100 Einheiten/kg Körpergewicht pro Verabreichung verabreicht wird.

6. Verbindung zur Verwendung nach einem der vorhergehenden Ansprüche, wobei das trächtige Säugetier ein schwangerer Mensch ist

7. Verbindung zur Verwendung nach einem der vorhergehenden Ansprüche, wobei bei dem trächtigen Säugetier durch Messung von Inositolphosphoglykanen vom P-Typ (IPG vom P-Typ) in einer Körperflüssigkeit, wie z.B. in Blut oder Urin, ein Präeklampsierisiko diagnostiziert wird.

8. Verbindung zur Verwendung nach einem der vorhergehenden Ansprüche, wobei bei dem trächtigen Säugetier durch Messung von aus der Plazenta stammenden Inositolphosphoglykanen vom P-Typ (IPG vom P-Typ) in einer Körperflüssigkeit, wie z.B. in Blut oder Urin, ein Präeklampsierisiko diagnostiziert wird.

## Revendications

1. Composé pour l'utilisation dans la prévention de la pré-éclampsie chez une mammifère enceinte présentant un risque de pré-éclampsie, où ledit composé est un inhibiteur de la C1 estérase et où le composé est administré à une dose allant de 25 unités/kg de poids corporel à 100 unités/kg de poids corporel par administration à la mammifère enceinte au moins deux fois par semaine.

2. Composé pour l'utilisation selon la revendication 1, où l'inhibiteur de la C1 estérase est un inhibiteur recombinant de la C1 estérase, tel qu'un inhibiteur de la C1 estérase ayant une séquence d'acides aminés qui est sensiblement identique à la séquence d'acides aminés de l'inhibiteur de la C1 estérase dérivé du plasma humain.

3. Composé pour l'utilisation selon la revendication 2, où l'inhibiteur recombinant de la C1 estérase est produit chez un mammifère non humain transgénique, tel qu'une souris, une chèvre, un bovin, un mouton, un porcin ou un animal de l'ordre des *Lagomorpha,* tel qu'un *Leporidae,* incluant un lapin.

4. Composé pour l'utilisation selon la revendication 3, où l'inhibiteur de la C1 estérase a un niveau réduit de résidus d'acide sialique terminaux par rapport à l'inhibiteur de la C1 estérase dérivé du plasma, où ledit niveau réduit de résidus d'acide sialique terminaux résulte en une demi-vie plasmatique de moins de 6 heures.

5. Composé pour l'utilisation selon l'une quelconque des revendications 1 à 4, où le composé est administré à une dose allant de 50 unités/kg de poids corporel à 100 unités/kg de poids corporel par administration.

6. Composé pour l'utilisation selon l'une quelconque des revendications précédentes, où la mammifère enceinte est une humaine enceinte.

7. Composé pour l'utilisation selon l'une quelconque des revendications précédentes, où la mammifère enceinte est diagnostiquée comme étant à risque de pré-éclampsie par la mesure des inositolphosphoglycanes de type P (IPG de type P) dans un fluide corporel, tel que dans le sang ou l'urine.

8. Composé pour l'utilisation selon l'une quelconque des revendications précédentes, où la mammifère enceinte est diagnostiquée comme étant à risque de pré-éclampsie par la mesure des inositolphosphoglycanes de type P dérivés du placenta (IPG de type P) dans un fluide corporel, tel que dans le sang ou l'urine.
